(19) Europäisches Patentamt
European Patent Office
Office européen des brevets

(11) Veröffentlichungsnummer: **0 021 103**
**B1**

(12) # EUROPÄISCHE PATENTSCHRIFT

(45) Veröffentlichungstag der Patentschrift: **25.05.83**

(51) Int. Cl.[3]: **C 07 D 473/08**, A 61 K 31/52

(21) Anmeldenummer: **80103002.4**

(22) Anmeldetag: **29.05.80**

(54) **Theophyllin-Derivate, Verfahren zu ihrer Herstellung und diese Verbindungen enthaltende Arzneimittel.**

(30) Priorität: **31.05.79 DE 2922159**

(43) Veröffentlichungstag der Anmeldung: **07.01.81 Patentblatt 81/1**

(45) Bekanntmachung des Hinweises auf die Patenterteilung: **25.05.83 Patentblatt 83/21**

(84) Benannte Vertragsstaaten: **AT BE CH DE FR GB IT LI LU NL SE**

(56) Entgegenhaltungen:
**EP-A-0 011 399**
**DE-A-2 550 000**
**DE-A-2 804 416**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Friebe, Walter-Gunar, Dr. rer. nat., Heidelberger Landstrasse 111d, D-6100 Darmstadt (DE)**
Erfinder: **Thiel, Max, Dr. rer. nat., S 6, 35, D-6800 Mannheim 1 (DE)**
Erfinder: **Kampe, Wolfgang, Dr. rer. nat., Zedernstrasse 49, D-6805 Heddesheim (DE)**
Erfinder: **Wilhelms, Otto-Henning, Dr. rer. nat., Odenwandstrasse 25/2, D-6941 Weinheim-Rittenweier (DE)**
Erfinder: **Roesch, Androniki, Dr. med., Am Oberen Luisenpark 22, D-6800 Mannheim 1 (DE)**

**EP 0 021 103 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Theophyllin-Derivate, Verfahren zu ihrer Herstellung und diese Verbindungen enthaltende Arzneimittel

In der DE-A1-2 804 416 sind Purinderivate mit antiallergischer, entzündungswidriger und antiödematöser Wirkung beschrieben, die sich von Adenin bzw. am in 6-Stellung befindlichen Stickstoffatom substituierten Adeninderivaten ableiten. Es wurde nun gefunden, daß ähnliche Derivate des Theophyllins allergische Bronchialobstruktionen in niedrigen Dosen hemmen, allergische Hautreaktionen auch nach oraler Gabe abschwächen und Antihistamineffekte zeigen. Die in DE-A1-2 550 000 beschriebenen Purinderivate wirken antiallergisch, entzündungswidrig, antiödematös und außerdem antihypertensiv.

Die vorliegende Erfindung betrifft daher Theophyllin-Derivate der allgemeinen Formel I

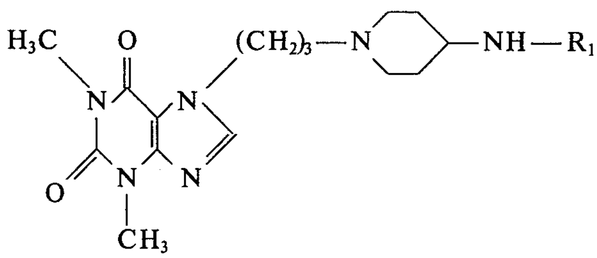

(I)

in der

$R_1$ einen $C_{1-8}$-Alkanoyl-Rest, der ein- oder mehrfach durch Phenyl substituiert sein kann,
einen $C_{1-8}$-Alkenoyl-Rest, der durch Phenyl substituiert ist,
einen Benzoyl-Rest, der ein- oder mehrfach durch Fluor, Chlor, Brom, Hydroxyl, $C_1-C_6$-Alkyl, $C_1-C_6$-Alkoxy, Alkoxycarbonyl mit 1 – 7 C-Atomen, $C_1-C_6$-Acyloxy, Carboxyl, Nitro, Amino, Nitril, Trifluormethyl, Carbamoyl oder Benzyl substituiert sein kann,
einen Furoyl-, Thenoyl- oder Pyridincarbonyl-Rest oder einen Cycloalkylcarbonyl-Rest mit 3 – 7 Ringkohlenstoffatomen

bedeutet sowie deren pharmakologisch verträgliche Salze.

Weiterhin betrifft die Erfindung pharmazeutische Präparate mit einem Gehalt an Verbindungen der allgemeinen Formel I.

Als Alkenoylrest in der Definition von $R_1$ ist besonders die Cinnamoylgruppe bevorzugt.

Außer den in den Beispielen genannten Verbindungen sind Gegenstand der vorliegenden Erfindung insbesondere alle Substanzen, die jede mögliche Kombination der in den Beispielen genannten Substituenten aufweisen.

Das Verfahren zur Herstellung der erfindungsgemäßen Verbindungen der Formel I ist dadurch gekennzeichnet, daß man in an sich bekannter Weise Theophyllin (Formel II)

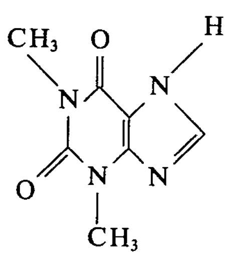

(II)

mit einer Verbindung der allgemeinen Formel III

$$X-CH_2-CH_2-CH_2-Y \qquad (III)$$

in der

X und Y jeweils Chlor, Brom, Mesyloxy oder Tosyloxy bedeuten,
und einer Verbindung der allgemeinen Formel IV

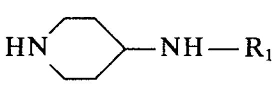

(IV)

in der

$R_1$ die obengenannte Bedeutung hat,

umsetzt,

anschließend gewünschtenfalls die Gruppe $R_1$ durch Verseifung und/oder folgende Acylierung mit einer Verbindung $R_1-Z$, wobei Z einen reaktiven Rest bedeutet, gegen eine andere Gruppe $R_1$ austauscht, gegebenenfalls einen Nitro-Substituenten des Benzoyl-Restes durch Hydrierung in eine Aminogruppe überführt und anschließend das erhaltene Reaktionsprodukt gewünschtenfalls in ein pharmakologisch verträgliches Salz überführt.

Reaktive Reste Z können alle Reste sein, die in der Peptid-Chemie zur Aktivierung von Carbonsäuren Verwendung finden, beispielsweise Halogenatome, die Azido-Gruppe, Alkyloxy-, Aryloxy- und Acyloxy-Gruppen.

Das erfindungsgemäße Verfahren führt man beispielsweise so durch, daß man zunächst Verbindungen der allgemeinen Formel III mit Verbindungen der allgemeinen Formel IV kondensiert und das erhaltene Reaktionsprodukt isoliert. Dieses Zwischenprodukt wird dann mit Theophyllin (II) zur Reaktion gebracht. Die Reaktion erfolgt zweckmäßig in alkalischem Medium, vorzugsweise in einem niederen Alkohol, wie z. B. in Isopropanol, in Anwesenheit von Natriumisopropanolat.

Eine andere Variante besteht darin, zunächst Theophyllin (II) mit Verbindungen der allgemeinen Formel III zur Reaktion zu bringen; anschließend wird das erhaltene Reaktionsgemisch mit Verbindungen der allgemeinen Formel IV zum gewünschten Endprodukt der allgemeinen Formel I umgesetzt.

Eine nachträgliche Umwandlung der Gruppe $R_1$ in der allgemeinen Formel I in eine andere Gruppe $R_1$ erfolgt beispielsweise als Austausch eines Alkanoyl-, Alkenoyl-, Benzoyl-, Furoyl-, Thenoyl-, Pyridincarbonyl- oder Cycloalkylcarbonyl-Restes $R_1$ gegen einen anderen Rest $R_1$. Hierzu werden Verbindungen der Formel I zunächst in saurem Milieu verseift und die erhaltenen Zwischenprodukte vorzugsweise in Gegenwart eines säurebindenden Mittels nach bekannten Methoden acyliert. Die als Zwischenprodukt erhaltenen [3-(4-Amino-piperidino)-propyl]-xanthin-Derivate sind ebenfalls neue Verbindungen.

Des weiteren kann in Verbindungen der Formel I, in denen der Acylrest $R_1$ eine Nitro-Gruppe enthält, die Nitro-Gruppe durch bekannte Methoden in eine Amino-Gruppe umgewandelt werden, z. B. durch katalytische Hydrierung.

Theophyllin (II) ist literaturbekannt. Die Verbindungen der Formeln III und IV sind aus der Literatur bekannt oder können von bekannten Verbindungen ausgehend nach trivialen Methoden leicht hergestellt werden.

Die pharmakologisch verträglichen Salze erhält man in üblicher Weise, z. B. durch Neutralisation der Verbindungen der Formel I mit nichttoxischen anorganischen oder organischen Säuren, wie z. B. Salzsäure, Schwefelsäure, Phosphorsäure, Bromwasserstoffsäure, Essigsäure, Milchsäure, Zitronensäure, Äpfelsäure, Salicylsäure, Malonsäure, Maleinsäure und Bernsteinsäure.

Die erfindungsgemäßen neuen Substanzen der Formel I und ihre Salze können in flüssiger oder fester Form enteral und parenteral appliziert werden. Hierbei kommen alle üblichen Applikationsformen in Frage, beispielsweise Tabletten, Kapseln, Dragees, Sirupe, Lösungen, Suspensionen etc. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z. B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

In den folgenden Beispielen wird die Herstellung der erfindungsgemäßen Verbindungen sowie einer pharmazeutischen Zubereitungsform näher beschrieben.

## Beispiel 1

7-⟨3-[4-(4-Fluor-benzamido)-piperidino]-propyl⟩-theophyllin

Zu der Lösung von 0,46 g (0,02 mol) Natrium in 100 ml Isopropanol gibt man 3,6 g (0,02 mol) Theophyllin, erhitzt 10 min am Rückflußkühler, kühlt ab und fügt 6,5 g (0,022 mol) 3-[4-(4-Fluor-benzamido)-piperidino]-propylchlorid zu. Nach 6 Stunden Rühren unter Rückfluß läßt man abkühlen, filtriert, wäscht den Niederschlag mit 2 N Natronlauge und Wasser und kristallisiert aus Ethylacetat um. Man erhält 5,8 g 7-⟨3-[4-(4-Fluor-benzamido)-piperidino]-propyl⟩-theophyllin (66% d. Th.) vom Schmp. 192 – 193° C.

## Beispiel 2

In analoger Weise wie in Beispiel 1 beschrieben erhält man:

| | Bezeichnung | Ausbeute % | Schmelzpunkt °C (Lösungsmittel) |
|---|---|---|---|
| a) | 7-[3-(4-Benzamido-piperidino)-propyl]-theophyllin aus Theophyllin und 3-(4-Benzamido-piperidino)-propylchlorid | 42 | 177–178 (Essigester/Ligroin) |
| b) | 7-{3-[4-(4-t-Butyl-benzamido)-piperidino]-propyl}-theophyllin aus Theophyllin und 3-[4-(4-t-Butyl-benzamido)-piperidino]-propylchlorid | 47 | 163–165 (Essigester/Ligroin) |
| c) | 7-[3-(4-Acetamido-piperidino)-propyl]-theophyllin aus Theophyllin und 3-(4-Acetamido-piperidino)-propylchlorid | 25 | 155–157 (Essigester/Ligroin) |
| d) | 7-{3-[4-(2-Methyl-benzamido)-piperidino]-propyl}-theophyllin aus Theophyllin und 3-[4-(2-Methyl-benzamido)-piperidino-propylchlorid | 37 | 182–183 (Isopropanol) |
| e) | 7-[3-(4-Phenylacetamido-piperidino)-propyl]-theophyllin aus Theophyllin und 3-(4-Phenylacetamido-piperidino)-propylchlorid | 36 | 158–160 (Methanol/Ether) |
| f) | 7-{3-[4-(2-Methoxy-benzamido)-piperidino]-propyl}-theophyllin-hydrochlorid aus Theophyllin und 3-[4-(2-Methoxy-benzamido)-piperidino]-propylchlorid | 38 | 254–255 (Methanol) |

## Beispiel 3

7-⟨3-[4-(Furan-2-carbonylamido)-piperidino]-propyl⟩-theophyllin

Zu einer Mischung aus 8,0 g (0,025 mol) 7-[3-(4-Amino-piperidino)-propyl]-theophyllin und 100 ml 1 N Natronlauge sowie 50 ml Tetrahydrofuran tropft man die Lösung von 7,8 g (0,06 mol) Furan-2-carbonylchlorid in 30 ml Tetrahydrofuran, rührt 5 Stunden bei Raumtemperatur, filtriert und kristallisiert aus Methanol um.

Man erhält 5,0 g 7-⟨3-[4-(Furan-2-carbonylamido)-piperidino]-propyl⟩-theophyllin (49% d. Th.) vom Schmp. 195 – 196° C.

Das als Reaktionskomponente verwendete 7-[3-(4-Amino-piperidino)-propyl]-theophyllin kann wie folgt erhalten werden:

Eine Mischung aus 15,0 g (0,1 mol) 4-Oximinopiperidin-Hydrochlorid, 55 ml (0,4 mol) Triethylamin und 11 ml (0,11 mol) 1-Brom-3-chlor-propan wird 16 Stunden unter Rückfluß erhitzt, abgekühlt, filtriert und das Filtrat eingeengt. Es verbleiben 15,8 g 3-(4-Oximino-piperidino)-propylchlorid (84% d. Th.) als Öl.

Zu der Lösung von 3,4 g (0,15 mol) Natrium in 350 ml Isopropanol fügt man 26,6 g (0,15 mol) Theophyllin, erhitzt 10 min unter Rückfluß, fügt 28,0 g (0,15 mol) 3-(4-Oximino-piperidino)-propylchlorid zu, erhitzt 16 Stunden unter Rückfluß, engt ein, nimmt den Rückstand in Methylenchlorid auf,

wäscht mit Wasser und engt ein. Nach Verreiben mit Ether verbleiben 28,5 g 7-[3-(4-Oximino-piperidino)-propyl]-theophyllin (57% d. Th.) vom Schmp. 167 – 170° C.

Eine Lösung von 6,7 g (0,02 mol) der vorstehend genannten Verbindung in 150 ml Methanol und 150 ml Tetrahydrofuran hydriert man unter Zusatz von 5 ml Raney-Nickel bei Raumtemperatur und 1 bar Wasserstoffdruck. Nach Beendigung der Wasserstoffaufnahme wird filtriert und eingeengt. Es verbleiben 6,3 g 7-[3-(4-Amino-piperidino)-propyl]-theophyllin (90% d. Th.) als Öl.

## Beispiel 4

In analoger Weise wie in Beispiel 3 beschrieben erhält man:

| | Bezeichnung | Ausbeute % | Schmelzpunkt °C (Lösungsmittel) |
|---|---|---|---|
| a) | 7-{3-[4-(2-Hydroxy-benzamido)-piperidino]-propyl}-theophyllin aus 7-[3-(4-Amino-piperidino)-propyl]-theophyllin und Salicylsäurechlorid | 38 | 210–213 (Isopropanol) |
| b) | 7-[3-(4-Cyclopropancarbonylamido-piperidino)-propyl]-theophyllin aus 7-[3-(4-Amino-piperidino)-propyl]-theophyllin und Cyclopropancarbonsäurechlorid | 42 | 182–183 (Methanol) |
| c) | 7-{3-[4-Nitro-benzamido)-piperidino]-propyl}-theophyllin aus 7-[3-(4-Amino-piperidino)-propyl]-theophyllin und 4-Nitrobenzoylchlorid | 44 | 220–222 (Methanol) |
| d) | 7-{3-[4-(Thiophen-2-carboxamido)-piperidino]-propyl}-theophyllin aus 7-[3-(4-Amino-piperidino)-propyl]-theophyllin und Thiophen-2-carbonylchlorid | 68 | 246–248 (Methanol) |

## Beispiel 5

7-⟨3-[4-(4-Amino-benzamido)-piperidino]-propyl⟩-theophyllin

Eine Mischung aus 4,8 g (0,01 mol) 7-⟨3-[4-(4-Nitro-benzamido)-piperidino]-propyl⟩-theophyllin (Beispiel 4c), 150 ml Methanol, 150 ml Tetrahydrofuran und 3 ml Raney-Nickel wird bei Raumtemperatur und 1 bar Wasserstoffdruck hydriert. Danach wird filtriert, eingeengt, der Rückstand in verd. Salzsäure aufgenommen, mit Ether extrahiert, alkalisch gestellt und filtriert. Es verbleiben nach Umkristallisation aus Methanol 2,7 g 7-⟨3-[4-(4-Amino-benzamido)-piperidino]-propyl⟩-theophyllin (60% d. Th.) vom Schmp. 203 – 205° C.

## Beispiel 6

7-[3-(4-Benzamido-piperidino)-propyl]-theophyllin-hydrochlorid

Eine Lösung von 1,0 g 7-[3-(4-Benzamido-piperidino)-propyl]-theophyllin (Beispiel 2a) in 20 ml Ethylacetat versetzt man mit überschüssiger etherischer Chlorwasserstofflösung, filtriert und wäscht den Niederschlag mit Aceton und Ether. Man erhält 0,95 g 7-[3-(4-Benzamido-piperidino)-propyl]-theophyllin-hydrochlorid (88% d. Th.) vom Schmp. oberhalb 290° C.

Beispiel 7

| | Wirkstoffhaltige Tabletten | für 1 Tabl. | für 100 000 Tabl. |
|---|---|---|---|
| I | Wirkstoff | | |
| | 7-{3-[4-(2-Hydroxy-benzamido)-piperidino]-propyl}-theophyllin | 10,000 mg | 1,000 kg |
| | Lactose | 67,000 mg | 6,700 kg |
| | Maisstärke | 35,000 mg | 3,500 kg |
| II | Polyvinylpyrrolidon, Mol.-Gew. 30.000 | 3,000 mg | 0,300 kg |
| III | Natriumcarboxymethylamylopektin | 4,000 mg | 0,400 kg |
| | Cellulosepulver | 20,000 mg | 2,000 kg |
| | Magnesiumstearat | 1,000 mg | 0,100 kg |
| | | 140,000 mg | 14,000 kg |
| | Wasser zum Granulieren | | 1,000 kg |

Herstellung:

Die Substanzen I werden mit der wäßrigen Lösung aus II granuliert, getrocknet und gesiebt. Das Granulat wird mit den Substanzen III zur Tablettiermasse gemischt. Die Tablettierung erfolgt zu Tabletten von 7 mm Durchmesser und 140 mg Gewicht.

Versuchsprotokoll

Methode: (nach KONZETT-RÖSSLER 133 a)

Die Versuchsanordnung zur Messung des Bronchospasmus entsprach im Prinzip der von KONZETT und RÖSSLER (1940) in der Modifikation nach COLLIER u. a. (1960). Tieren in mit 40 mg/kg Pentobarbital-Natrium i. p. herbeigeführter Narkose wurde eine Y-Kanüle in die Trachea und eine Venenkanüle in die V. jugularis eingebunden. Eine in die A. carotis eingebundene Kanüle führte zu einer Meßkammer und der arterielle Druck wurde über eine Gleichspannungsmeßbrücke aufgezeichnet. Mit der Atempumpe wurde den Ratten ein Luftvolumen von je nach Größe der Tiere 1 – 2 ml 64mal, den Meerschweinchen von 7 – 15 ml 72mal pro Minute zugeführt. Die Narkose mußte so tief sein, daß eine spontane Gegenatmung von Seiten der Tiere nicht auftrat. Bei Auftreten eines Bronchospasmus konnte bei gleichbleibendem Beatmungsvolumen das gesamte angebotene Luftvolumen nicht mehr in die Lunge strömen und floß teilweise durch einen Seitenarm über ein Ventil ab. Die von der Firma BASILE entwickelte Methode weicht insofern von der ursprünglichen von KONZETT und RÖSSLER beschriebenen Methode ab, als dieses Luftvolumen indirekt als Strömungsgeschwindigkeit mit Hilfe eines Thermoelementes gemessen wird. Jeder Bronchospasmus konnte dann als prozentuale Abnahme des verfügbaren Bronchialvolumens beschrieben werden. Den theoretisch maximal möglichen Bronchospasmus erhielt man durch Abklemmen des Beatmungsschlauches am Tier, so daß die gesamte zugeführte Luft durch das Ventil abströmte.

Alle 30 sec wurde durch ein Relais das Ventil für 8 sec geschlossen und das gesamte Volumen in die Lunge gepumpt, um die Bronchen und Alveolen wieder zu entfalten und so einen ausreichenden Gasaustausch zu ermöglichen. Die prozentuale Abnahme des verfügbaren Bronchialvolumens (X) wurde nach folgender Formel errechnet:

$$X = \frac{b-a}{m-a} \times 100$$

a = Ausschlag des Schreibers bei Versuchsbeginn in mm.
b = Höhe des Ausschlages zu einem bestimmten Zeitpunkt in mm.
m = Maximaler Ausschlag in mm nach Abklemmen des Beatmungsschlauches am Tier.

COLLIER, H. O. J., J. A. HOLGATE, M. SCHACHTER: THE BRONCHOCONSTRICTOR ACTION OF BRADYKININ IN THE GUINEA-PIG. Brit. J. Pharmacol. 15, 290 (1960).

KONZETT, H. und R. RÖSSLER: Versuchsanordnung zu Untersuchungen an der Bronchialmuskulatur Naunyn-Schmiedebergs Arch. exp. Path. Pharmak. 195, 71 – 74 (1940).

Präparation des Antiserums

Das Antigen ist 2× kristallisiertes Ovalbumin. Gleiche Mengen einer Lösung des Antigens in physiologischer Kochsalzlösung und komplettem Freund'schen Adjuvans werden emulsiert und 2× 0,15 ml i. m. bei männlichen Meerschweinchen injiziert. Die Tiere werden entblutet und das gepoolte Serum bei −20°C aufbewahrt. Zur passiven Sensibilisierung werden Meerschweinchen 0,5 ml des Antiserums 1 : 50 verdünnt 24 – 48 Stunden vor Auslösen des Bronchospasmus i. v. injiziert.
(DAVIES und JOHNSON: Int. Arch. Allergy 41, 648 – 654 [1971]).

Inhibierung der antigen-induzierten Bronchospasmen (BR Sp) in passiv sensibilisierten Meerschweinchen

| Beispiel Nr. | % Inhibierung Br Sp | |
|---|---|---|
| | Dosis mg/kg i.V. | % |
| 2c | 0.38 | 37 |
| 2d | 0.1 | 60 |
| 2e | 0.38 | 68 |
| 4a | 0.1 | 82 |
| 4d | 0.1 | 38 |
| Aminophyllin*) | 24 | 29 |

*) Aminophyllin = 2 Mol Theophyllin + 1 Mol Ethylendiamin

**Patentansprüche**

1. Theophyllin-Derivate der allgemeinen Formel I

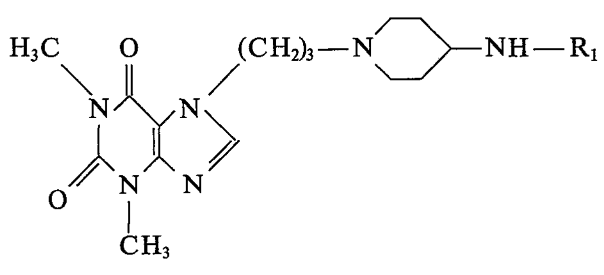

(I)

in der

$R_1$ einen $C_{1-8}$-Alkanoyl-Rest, der ein- oder mehrfach durch Phenyl substituiert sein kann,
einen $C_{1-8}$-Alkenoyl-Rest, der durch Phenyl substituiert ist,
einen Benzoyl-Rest, der ein- oder mehrfach durch Fluor, Chlor, Brom, Hydroxyl, $C_1-C_6$-Alkyl, $C_1-C_6$-Alkoxy, Alkoxycarbonyl mit 1 – 7 C-Atomen,
$C_1-C_6$-Acyloxy, Carboxyl, Nitro, Amino,

Nitril, Trifluormethyl, Carbamoyl oder Benzyl substituiert sein kann,
einen Furoyl-, Thenoyl- oder Pyridincarbonyl-Rest oder einen Cycloalkylcarbonyl-Rest mit 3–7 Ringkohlenstoffatomen

bedeutet sowie deren pharmakologisch verträgliche Salze.

2. 7-[3-(4-Amino-piperidino)-propyl]-theophyllin sowie deren Salze mit anorganischen oder organischen Säuren.

3. Verfahren zur Herstellung von Theophyllin-Derivaten gemäß Anspruch 1, dadurch gekennzeichnet, daß man in an sich bekannter Weise Theophyllin (II) mit einer Verbindung der allgemeinen Formel III

$$X-CH_2-CH_2-CH_2-Y \quad (III)$$

in der

X und Y jeweils Chlor, Brom, Mesyloxy oder Tosyloxy bedeuten,
und einer Verbindung der allgemeinen Formel IV

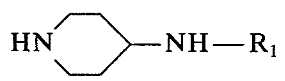

(IV)

in der

$R_1$ die obengenannte Bedeutung hat,

umsetzt,

anschließend gewünschtenfalls die Gruppe $R_1$ durch Verseifung und/oder folgende Acylierung mit einer Verbindung $R_1-Z$, wobei Z einen reaktiven Rest bedeutet, gegen eine andere Gruppe $R_1$ austauscht, gegebenenfalls einen Nitro-Substituenten des Benzoyl-Restes durch Hydrierung in eine Aminogruppe überführt und anschließend das erhaltene Reaktionsprodukt gewünschtenfalls in ein pharmakologisch verträgliches Salz überführt.

4. Arzneimittel, enthaltend einen Wirkstoff gemäß Anspruch 1 sowie an sich bekannte pharmakologisch verträgliche Träger- und Hilfsstoffe.

5. Verbindungen gemäß Anspruch 1 zur Anwendung bei der Behandlung von Allergien.

**Claims**

1. Theophyllin derivatives of the general formula I

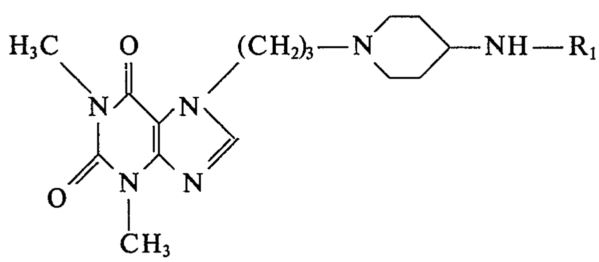

(I)

in which $R_1$ signifies a $C_{1-8}$ alkanoyl radical, which can be substituted one or more times by phenyl; a $C_{1-8}$ alkenoyl radical, which is substituted by phenyl; a benzoyl radical, which can be substituted by one or more times by fluorine, chlorine, bromine, hydroxyl, $C_1-C_6$ alkyl, $C_1-C_6$ alkoxy, alkoxycarbonyl with 1–7 C-atoms, $C_1-C_6$ acyloxy, carboxyl, nitro, amino, nitrile, trifluoromethyl, carbamoyl or benzyl; a furoyl, thenoyl or pyridinecarbonyl radical or a cycloalkylcarbonyl radical with 3–7 ring carbon atoms, as well as their pharmacologically acceptable salts.

2. 7-[3-(4-Aminopiperidino)-propyl]-theophyllin, as well as its salts with inorganic or organic acids.

3. Process for the preparation of theophyllin derivatives according to claim 1, characterised in that in per se known manner one reacts theophyllin (II) with a compound of the general formula III

$$X-CH_2-CH_2-CH_2-Y \quad (III)$$

in which X and Y each signify chlorine, bromine, mesyloxy or tosyloxy, and a compound of the general formula IV

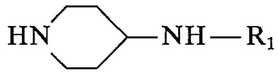

(IV)

in which $R_1$ has the above-mentioned meaning, subsequently, if desired, exchanges the group $R_1$ by saponification and/or subsequent acylation with a compound $R_1-Z$, whereby Z signifies a reactive residue, for another group $R_1$, optionally converts a nitro substituent of the benzoyl radical by hydrogenation into an amino group and subsequently, if desired, converts the reaction product obtained into a pharmacologically acceptable salt.

4. Medicaments containing an active material according to claim 1, as well as per se known pharmacologically acceptable carriers and adjuvants.

5. Compounds according to claim 1 for use in the treatment of allergies.

**Revendications**

1. Dérivés de théophylline de formule générale I

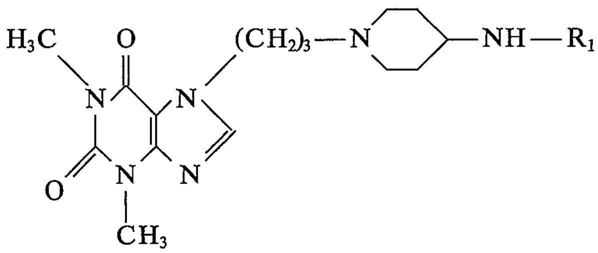

(I)

dans laquelle

$R_1$ est un reste alcanoyle $C_{1-8}$, pouvant être substitué une ou plusieurs fois par un groupe phényle, un reste alcénoyle $C_{1-8}$, substitué par une groupe phényle, un reste benzoyle pouvant être substitué une ou plusieurs fois par le fluor, le chlore, le brome, un groupe hydroxyle, alkyle $C_1-C_6$, alcoxy $C_1-C_6$, alcoxy carbonyle ayant de 1 à 7 atomes de carbone, acyloxy $C_1-C_6$, carboxyle, nitro, amino, nitrile, trifluorométhyle, carbamoyle ou benzyle, un reste furoyle, thénoyle ou pyridine-carbonyle, ou un reste cyclo-alkylcarbonyle ayant de 3 à 7 atomes de carbone dans le noyau,

ainsi que leurs sels pharmacologiquement tolérables.

2. La 7-[3-(4-amino-pipéridino)propyl]théophylline ainsi que leurs sels formés avec des acides minéraux ou organiques.

3. Procédé pour la préparation de dérivés de théophylline selon la revendication 1, caractérisé en ce qu'on fait réagir de façon en soi connue la théophylline (II) avec un composé de formule générale III

$$X-CH_2-CH_2-CH_2-Y \quad (III)$$

dans laquelle

X et Y désignent chacun le chlore, le brome, le mésyloxy ou le tosyloxy, et avec un composé de formule générale IV

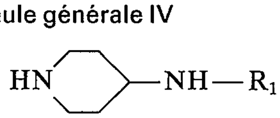

(IV)

dans laquelle

$R_1$ a la signification ci-sessus donnée,

et on échange ensuite éventuellement le groupe $R_1$ par estérification et/ou acylation consécutive avec un composé $R_1-Z$, où Z est un reste réactif, contre un autre groupe $R_1$, on transforme éventuellement un substituant nitro du reste benzoyle par hydrogénation en un groupe amino et ensuite on transforme éventuellement le produit de réaction obtenu en un sel pharmacologiquement tolérable.

4. Médicament, contenant une substance active selon la revendication 1 ainsi que des substances de support et auxiliaires pharmacologiquement tolérables en soi connues.

5. Composés selon la revendication 1 destinés à être mis en oeuvre pour le traitement d'allergies.